Europäisches Patentamt

European Patent Office

Office européen des brevets

(11) Numéro de publication: **0 274 946 B1**

# FASCICULE DE BREVET EUROPEEN

(45) Date de publication de fascicule du brevet: **08.04.92**  (51) Int. Cl.5: **A23J 3/00**

(21) Numéro de dépôt: **87402837.6**

(22) Date de dépôt: **14.12.87**

(54) **Procédé de fabrication d'un hydrolysat enzymatique de protéines riche en di-et tri-peptides, utilisable notamment en nutrition artificielle et en diététique.**

(30) Priorité: **15.12.86 FR 8617516**

(43) Date de publication de la demande:
**20.07.88 Bulletin 88/29**

(45) Mention de la délivrance du brevet:
**08.04.92 Bulletin 92/15**

(84) Etats contractants désignés:
**AT BE CH DE ES FR GB GR IT LI LU NL SE**

(56) Documents cités:
**FR-A- 2 380 295**
**US-A- 3 857 966**
**US-A- 3 970 520**

(73) Titulaire: **PHARMACIA FRANCE S.A.**
**1 Rue Antoine Lavoisier**
**78280 GUYANCOURT(FR)**

(72) Inventeur: **Chataud, Jean**
**Route de Chinon Noyant de Touraine**
**F-37800 Sainte Maure de Touraine(FR)**
Inventeur: **Desreumaux, Serge**
**La Cholleterie**
**F-37250 Veigne(FR)**
Inventeur: **Cartwright, Terence**
**Le Moulin de l'Etang**
**F-37250 Saint Epain(FR)**

(74) Mandataire: **Portal, Gérard et al**
**Cabinet Beau de Loménie 55, rue d'Amsterdam**
**F-75008 Paris(FR)**

**Description**

La présente invention concerne un procédé simple et économique de fabrication d'un hydrolysat enzymatique de protéines riche en di- et tri-peptides, utilisable notamment en nutrition aritificelle et en diététique.

Il est de nos jours admis et démontré que les di- et tri-peptides présentent un réel intérêt en nutrition artificielle et en diététique.

La nutrition artificielle est une technique appliquée aux patients incapables de s'alimenter par la voie normale en raison de dommages physiques (accident, opération chirurgicale, cancer de l'oesophage, etc) ou en raison d'un état physiologique général qui ne permet pas une alimentation normale (coma, infection sévère, trauma, brûlures, etc).

La nutrition artificielle peut être donnée par la voie naturelle (voie digestive), il s'agit alors de nutrition orale ou entérale. Dans d'autres cas, les nutriments sont introduits directement dans la circulation sanguine, il s'agit alors de nutrition parentérale.

Une nutrition complète nécessite l'administration de l'azote sous la forme de protides (protéines, peptides, aminoacides) en mélange avec des lipides, des hydrates de carbone, des éléments minéraux et des vitamines.

Dans une alimentation normale, la majeure partie de l'azote consommé est ingérée sous la forme de protéines et est digérée par les enzymes du tractus digestif.

Toutefois, dans un grand nombre de situations citées où la nutrition artificielle est utilisée, la digestion effective des protéines n'est pas possible du fait de déficiences enzymatiques dues aux maladies ou à la portion limitée de l'intestin restant fonctionnelle après la maladie ou l'intervention chirurgicale.

En nutrition orale et entérale, on utilise parfois des aminoacides purs comme apport protidique mais ceux-ci présentent toutefois de nombreux inconvénients théoriques et pratiques :

- les aminoacides sont transportés depuis l'intestin par des processus de transport actif. Différents aminoacides entrent en compétition pour le transport, et l'absorption intestinale peut être limitée par cette compétition ;
- certains états pathologiques peuvent perturber le transport de certains aminoacides, provoquant une réduction de la capacité d'absorption.

Il a été montré récemment (Silk D.B.A. Peptide absorption in man-Gut 15 194 (1974) ; Matthews D.M. Intestinal absorption of peptides - Phys. Rev. 55 537 (1975) ; Adibi S.A. et Y.S. Kim Peptide absorption and hydrolysis in Physiology of the gastrointestinal tract. Ed. L.R. Johnson, Raven Press New York 1073) que la molécule de peptide comprenant de 2 à 3 aminoacides liés par des liaisons peptidiques (di- ou tri-peptide respectivement) est absorbée à l'état intact à partir de l'intestin par un mécanisme distinct de celui mis en oeuvre dans le transport des aminoacides.

Il apparaît que les cellules de surface dans l'intestin absorbent les di- et tri-peptides, les hydrolysent in situ en aminoacides et libèrent les aminoacides ainsi obtenus dans le sang.

La digestion enzymatique des protéines a été largement utilisée comme un moyen de production de mélanges de peptides inférieurs ou "petits peptides".

Ainsi toutes les préparations décrites contiennent un large spectre de longueurs de chaîne peptidique (de 2 à plus de 20 aminoacides) mais la teneur en di- et tri-peptides spécialement requise est très faible (de l'ordre de 5 à 20 %).

Des peptides supérieurs aux di- et tri-peptides ne montrent pas les avantages de l'absorption cinétique décrite précédemment et ainsi ces mélanges de peptides mal définis ne produisent pas une absorption d'azote optimale dans les utilisations cliniques.

Il a également été proposé de préparer des hydrolysats de de protéines à l'aide d'enzymes utilisables en milieu acide, mais la digestion doit s'étendre sur une longue période de temps de 8 à 72 heures. Ces hydrolysats peuvent contenir des peptides dont le poids moléculaire est inférieur à 700 daltons. Toutefois, les enzymes utilisées ne sont pas d'un usage courant dans les industries alimentaires et diététiques et leur innocuité n'est pas unanimement reconnue. De plus, compte tenu de la durée de l'hydrolyse, celle-ci doit être conduite soit dans des conditions stériles (ce qui augmente nettement le coût de l'installation), soit en présence d'antiseptiques (dont l'élimination est difficile par la suite).

Ainsi, le document US-A-3.857.966 décrit un procédé d'hydrolyse enzymatique de matière protéique faisant intervenir un système comprenant une enzyme d'origine microbienne alcaline, une enzyme neutre et une enzyme végétale.

Le document FR-A-2.380.295 mentionne également la possibilité de préparer divers hydrolysats de protéines qui se différencient par la taille de leur molécule en utilisant des mélanges d'enzymes appropriés.

Ainsi, les hydrolysats enzymatiques de protéines classiques ne conviennent pas pour la production de mélanges contenant essentiellement des di- et tri-peptides.

On peut également préparer les di- et tri-peptides par synthèse chimique et l'expérience a montré que ces mélanges de di-peptides peuvent être utilisés d'une manière efficace dans la nutrition artificielle (FURST P. Peptides in parenteral nutrition - Clinical Nutrition 4 (Suppl. 1985) p. 105 ; KRZYSIK B.A. et ADIBI S.A. Comparison of metabolism of glycine injected intravenously in free and dipeptide forms ; STEINHARDT H.J., PALEOS G.A., BRANDL M., FEKL W.L., ADIBI S.A. - Efficacy of synthetic dipeptide mixture as the source of aminoacids for total parenteral nutrition in a subhuman primate).

Toutefois, cette approche présente également des inconvénients :

- en premier lieu, la totalité des di-peptides possibles (au nombre de 400) ne peut pas être raisonnablement synthétisée ; l'expérimentation était basée sur un nombre limité de di-peptides contenant la glycine (c'est-à-dire glycine - X, dans laquelle X est un autre aminoacide).

Cette approche limite les di-peptides disponibles et peut entraîner un déséquilibre dû à l'excès de glycine.

- en second lieu, une autre limite de l'approche par synthèse chimique est le coût de production des di-peptides qui est trop élevé pour un usage en nutrition.

La présente invention permet de remédier aux inconvénients des procédés classiques exposés ci-dessus, et concerne un procédé d'hydrolyse enzymatique des protéines pour la production des mélanges peptidiques contenant principalement des di- et tri-peptides.

Comme déjà mentionné ci-dessus, en alimentation orale ou entérale les di- et tri-peptides offrent plusieurs avantages par rapport aux nutriments ordinaires (aussi dénommés polymériques) et aux diètes élémentaires (qui contiennent les protides sous forme d'aminoacides).

Le premier avantage est d'ordre cinétique ; au niveau intestinal, l'apport azoté est plus rapide lorsque les protides sont sous forme de di- et tri-peptides que dans le cas où ceux-ci sont sous forme élémentaire ou oligomérique (produits de la digestion gastrique et pancréatique).

Un deuxième avantage tient à la non compétition au niveau de l'absorption qui existe entre les di- et tri-peptides d'une part et les aminoacides d'autre part.

Un troisième avantage est lié au faible rapport osmolarité/azote (deux à trois fois plus faible que celui des solutés d'aminoacides, ce qui permet d'avoir des solutés riches en azote et dont l'osmolarité n'est pas trop éloignée des valeurs physiologiques de 300 à 500 mOs/l.

On peut donc concevoir que dans certaines conditions pathologiques où les systèmes de transport des aminoacides sont perturbés, l'absorption des di- et tri-peptides peut ne pas être modifiée.

Le procédé selon l'invention comporte une étape d'hydrolyse enzymatique d'une protéine, à l'aide d'une association des trois enzymes suivantes, ajoutées simultanément ou successivement :

a) une protéase bactérienne utilisable en milieu voisin de la neutralité (pH 5 à 8), généralement extraite de souches de Bacillus subtilis, choisie dans le groupe comprenant la Neutrase® de la firme Novo Industrie Enzymes S.A., la Protéase B500® de la firme Rapidase Gist Brocadès, et la H.T. Protéolytique® de la firme Miles.

b) une protéase bactérienne utilisable en milieu alcalin (pH 7 à 11), extraite par exemple de souches de Bacillus licheniformis ou de souches de Bacillus subtilis, choisie dans le groupe comprenant l'Alcalase® de la firme Novo Industrie Enzymes S.A., la Protéase $A_2$® de la firme Rapidase Gist Brocadès, et l'Optimase® de la firme Miles,

c) une enzyme pancréatique d'origine animale, choisie dans le groupe comprenant la trypsine ou un concentré de trypsine ou la préparation commercialisée par la firme Novo Industrie Enzymes S.A. sous le nom de PEM®.

les conditions de l'hydrolyse enzymatique comprennent :

- un pH neutre à alcalin, de 7 à 10,
- une température de 20 à 70°C, de préférence de 30 à 50°C,
- une durée d'hydrolyse égale ou inférieure à 300 minutes.

La concentration de la matière protéique dans la suspension aqueuse à hydrolyser est de préférence de 5 à 20 % en poids par volume.

L'étape d'hydrolyse enzymatique est arrêtée par destruction de l'activité enzymatique, par exemple par un chauffage à 95-100°C pendant 10 à 15 minutes.

L'hydrolysat enzymatique de protéines ainsi obtenu est éventuellement purifié par ultrafiltration.

D'un point de vue industriel, les protéines utilisables pour la fabrication de cet hydrolysat comprennent à titre d'exemple les suivantes :

- blanc d'oeuf (ovalbumine)
- protéines laitières : lactalbumines et caséines
- protéines d'abattoirs : sérum albumine, hémoglobine décolorée

- produits de l'industrie des pêches et de la conserverie de poissons
- des produits d'origine végétale : protéines de soja de luzerne.

La Neutrase® Novo est présentée sous la forme d'un liquide brun clair, préparée à partir d'une culture purifiée de Bacillus subtilis; son activité protéolytique est de 0,5 unité Anson/g (une unité Anson est une unité enzymatique correspondant à la quantité d'enzyme qui libère dans les conditions expérimentales 1 mMole d'acides aminés Folin-positifs (exprimé en tyrosine) par minute.

L'Alcalase® Novo est présentée sous la forme d'un liquide brun foncé préparé par purification et concentration d'une culture de Bacillus licheniformis. Son activité protéolytique est soit de 0,6 unité Anson/g soit de 2,4 unités Anson/g.

La "P.E.M." est présentée sous la forme d'une poudre jaunâtre obtenue par un mélange standardisé de concentrés de trypsine d'origine porcine et bovine. Son activité protéolytique est :
- au moins égale à 1800 unités NF/USP pour l'activité trypsique,
- au moins égale à 350 unités NF/USP pour l'activité chymotrypsique,
- et le rapport trypsine/chymotrypsine est compris entre 3,75 et 6.

Le rapport enzyme/substrat est de 5-10 % pour l'"Alcalase" ou la "Neutrase" et de 0,5-1 % pour la PEM.

Le procédé selon l'invention peut être mis en oeuvre d'une façon économique dans la mesure où, il ne nécessite pas d'installations stériles et de matériel de centrifugation.

De plus, les enzymes sélectionnées sont toutes issues de microorganismes bien connus, déjà utilisés dans les industries alimentaires et dont l'innocuité ne fait aucun doute.

La présente invention est basée sur la découverte par les inventeurs d'une combinaison de protéases capable de dégrader les protéines en une sule étape, pour conduire directement à un mélange de protides de faible poids moléculaire et dont la composition est caractérisée par une forte teneur en di- et tripeptides, une faible teneur en aminoacides libres.

Les digestions enzymatiques, selon l'invention, sont conduites en milieu basique à neutre, l'apport en sodium et potassium est limité à la teneur souhaitée pour l'utilisation prévue, le délai de l'hydrolyse n'excède pas 300 minutes, de façon à éviter l'apparition d'une contamination bactérienne dans des conditions opératoires simples.

Comme déjà mentionné ci-dessus l'originalité de l'invention réside dans le choix d'une association de trois enzymes agissant successivement ou simultanément, de façon synergique et complémentaire.

Les essais suivants montrent la supériorité de cette association d'enzymes sur ces enzymes prises séparément ou associées deux par deux dans le cadre de l'hydrolyse enzymatique de protéines.

Le tableau I groupe les enzymes étudiées et les conditions d'hydrolyse (pH - température - concentration) dans lesquelles sont utilisées ces enzymes.

Le tableau II présente les résultats de 15 exemples d'hydrolyses enzymatiques réalisées soit sur le blanc d'oeuf de poule, soit sur la caséine du lait de vache sous forme de produits séchés par atomisation, en utilisant ces enzymes prises séparément, ou associées deux par deux ou trois par trois.

TABLEAU I

| Enzymes essayées et conditions d'hydrolyse utilisées. | | | | |
|---|---|---|---|---|
| enzymes | activité en unités | doses d'utilisation enzyme/substrat | °C des essais | zone d'utilisation pH |
| pepsine | 10 000 NF | 10g/kg | 35-45 | 1,8-2,5 |
| trypsine | 4 700 NF | 0,53g/kg | 40-50 | 7 à 9 |
| PEM [R] Novo | 2 500 NF | 10g/kg | 40-50 | 7 à 9 |
| Alcalase [R] Novo | 0,6 ANSON | 0,1l/kg | 40-50 | 7 à 9 |
| Neutrase [R] Novo | 0,5 ANSON | 0,1l/kg | 40-50 | 7 à 9 |

TABLEAU II

| N° essais | matières premières | ordre d'addition | | | degré d'hydrolyse (DH) % | aminoacides libres g/100g | tailles moyennes peptides |
|---|---|---|---|---|---|---|---|
| | | 1 | 2 | 3 | | | |
| 1 | blanc d'oeuf | Pepsine | | | 29 | 12 | 5,30 |
| 2 | b. d'oeuf | Trypsine | | | 10 | 2 | 12,00 |
| 3 | caséine | PEM® | | | 10 | 1 | 11,00 |
| 4 | caséine | Alcalase® | | | 36 | 12 | 3,70 |
| 5 | b. d'oeuf | Pepsine | Trypsine | | 34 | 15 | 4,50 |
| 6 | b. d'oeuf | Pepsine | Alcalase® | | 34 | 8 | 3,50 |
| 7 | b. d'oeuf | Pepsine | Alcalase® | Neutrase® | 37 | 12 | 3,50 |
| 8 | b. d'oeuf | Trypsine | Neutrase® | | 37 | 10 | 3,30 |
| 9 | b. d'oeuf | Trypsine | Alcalase® | | 36 | 9 | 3,30 |
| 10 | b. d'oeuf | Trypsine | Alcalase® | Neutrase® | 38 | 7 | 3,00 |
| 11 | b. d'oeuf | Trypsine | Neutrase® | Alcalase® | 38 | 7 | 3,00 |
| 12 | b. d'oeuf | Alcalase® | Neutrase® | PEM® | 38 | 7 | 3,00 |
| 13 | b. d'oeuf | Alcalase® | PEM® | | 33 | 4 | 3,30 |
| 14 | caséine | Acalase® | PEM® | | 33 | 4 | 3,30 |
| 15 | caséine | Alcalase® | Neutrase® | PEM® | 37 | 5 | 3,00 |

Le procédé particulier mise en oeuvre pour réaliser les 15 essais groupés dans le tableau II comporte les étapes suivantes :

a) mise en suspension de la matière première protéique dans l'eau distillée à la concentration 15 % (150 g/l) dans une cuve thermostatée munie d'un système d'agitation et de maintien du pH,

b) ajustement et maintien du pH à 8 et de la température à 45°C (pH 2,0 ; 40°C dans le cas des hydrolyses à la pepsine),

c) addition des préparations enzymatiques aux doses indiquées dans le tableau I,

d) hydrolyse pendant une durée de temps n'excèdant pas 300 minutes ; l'état d'avancement de l'hydrolyse est suivi par la quantité d'ions $OH^-$ utilisée (ions $H^+$ dans le cas de la pepsine) ; la réaction est arrêtée lorsque la dégradation souhaitée est atteinte.

e) destruction de l'activité enzymatique par chauffage à 100°C pendant 10 minutes,

f) ultrafiltration de l'hydrolysat et récupération de l'ultrafiltrat (point de coupure 10 000 ou 15 000 daltons).

L'hydrolysat ultrafiltré est analysé en vue de déterminer :

- le degré d'hydrolyse (ou son inverse, la taille moyenne des produits d'hydrolyse),
- le taux d'aminoacides libres,
- le taux de di- et tri-peptides dans l'hydrolysat ultrafiltré.

Le tableau II appelle les observations suivantes :

(1) l'utilisation d'une association de trois enzymes introduites simultanément dans le réacteur ou à quelques minutes d'intervalle conduit à un degré d'hydrolyse toujours supérieur à celui obtenu par une seule enzyme ou par une association de deux enzymes :

(2) en ce qui concerne les tailles moyennes des peptides obtenus, la nature des enzymes est déterminante ;

(3) dans certains cas, l'ordre d'addition des enzymes peut être permuté, sans que cela ait une incidence sur les produits obtenus (comparaison des essais 10 et 11). Toutefois, d'autres essais montrent que cet ordre pourrait être un élément important des conditions d'hydrolyse ;

(4) l'utilisation d'une troisième enzyme ne s'accompagne pas dans tous les cas d' une augmentation significative du taux d'aminoacides libres (comparaison des essais 8, 9 avec 10 et 14 avec 15) ;

(5) parmi ces différentes associations d'enzymes étudiées, une catégorie apparaît comme la plus performante tant en ce qui concerne le degré d'hydrolyse élevé que le faible taux d'aminoacides libres ; il s'agit de l'association : Alcalase® - Neutrase® - PEM® - (essais 12 et 15) et de l'association Trypsine - Alcalase® - Neutrase® - (essais 10 et 11) ;

(6) l'association : Alcalase®, Neutrase®, PEM® (ou Trypsine - Alcalase - Neutrase) est utilisable sur d'autres matières premières protéiques (sérum albumine bovine et porcine, protéines de soja et de luzerne, lactalbumine ou lait de vache, "hémoglobine décolorée" et produits de valorisation de l'industrie des pêches (déchets de poisson)). Dans tous ces cas, le degré d'hydrolyse est du même ordre de grandeur et le taux d'aminoacides libres est voisin de 5 %, sauf dans le cas où la matière première est déjà un hydrolysat (hémoglobine décolorée et produits de valorisation de l'industrie des pêches). Dans le cas de ces deux derniers exemples, le taux d'aminoacides libres est de l'ordre de 10 à 15 % en poids :

(7) les préparations enzymatiques PEM® - Alcalase® - Neutrase® peuvent être remplacées par d'autres préparations enzymatiques d'activité et de spécificité équivalentes fabriquées par d'autres firmes : Protéase B500® de la firme Rapidase Gist Brocadès - H.T. Protéolytique® de la firme Miles - Protéase A₂ de la firme Rapidase Gist Brocadès et Optimase® de la firme Miles ; (8) en ce qui concerne les essais 12 et 15, la concentration de la matière première protéique dans le milieu d'hydroloyse n'a pas de valeur critique.

Des essais effectués avec des concentrations comprises entre 5 et 20 % (en poids par volume) n'ont pas introduit de variations significatives du degré d'hydrolyse et de la taille des peptides, sous réserve que les préparations enzymatiques soient utilisées avec un rapport enzyme/substrat constant comme indiqué dans le tableau I.

Les hydrolysats protéiques sont généralement caractérisés par leur "degré d'hydrolyse" (D.H.), qui est la valeur en pourcentage du nombre de liaisons peptidiques rompues au nombre de liaisons peptidiques existantes dans la protéine native.

Le D.H. est approximativement l'inverse de la taille moyenne des produits de l'hydrolyse ; ainsi à un D.H. de 50 % correspond une taille moyenne de 2, et à un D.H. de 33 % une taille moyenne de 3.

La connaissance du D.H. et du taux d'aminoacides libres conduit à la détermination de la taille moyenne de peptides. Néanmoins, si le taux des aminoacides st faible, la taille moyenne des produits de l'hydrolyse et la taille moyenne des peptides sont pratiquement confondues.

6

Le D.H. (ou la taille moyenne des produits de l'hydrolyse) est calculé à partir de dosages classiques permettant la détermination du nombre de fonctions amines primaires avant et après l'hydrolyse chimique totale de l'hydrolysat enzymatique.

Le taux d'aminoacides libres est déterminé selon la technique AFNOR (NF V 76-115) adaptée aux hydrolysats enzymatiques de protéines.

L'évaluation du taux des di- et tri-peptides est déterminée par chromatographie analytique par échange de ligands. La phase stationnaire est constituée par du Sephadex-cuivre préparé selon la technique de ROTHENBUHLER E. (Anal. Biochem. 1979 : 97-387-75). La chromatographie s'effectue dans des conditions qui conduisent à une élution des oligopeptides par tailles moyennes décroissantes. On obtient ainsi sept fractions pour lesquelles on détermine une valeur moyenne (N) et le pourcentage de peptides de taille (N) contenu dans l'hydrolysat.

Les tableaux III et IV relatifs respectivement aux essais 12 et 15 montrent que les oligopeptides supérieurs aux tripeptides ont une taille moyenne au moins égale à cinq ce qui implique que la teneur en di- et tri-peptides est voisine ou supérieure à 50 % en poids.

## TABLEAU III

Hydrolyse du blanc d'oeuf par l'Alcalase®, la Neutrase® et la PEM®

| Etalonnage avec peptides synthétiques purs | N° fraction | TAILLES MOYENNES ET TENEURS | | |
| --- | --- | --- | --- | --- |
| | | Pour la fraction | Cumuls | |
| Cette fraction ne contient aucun tripeptide et aucun dipeptide | 1 | 8,5 ± 0,9 (12 %) | | |
| | | | 6,0 | |
| Ces fractions contiennent en majorité des tétrapeptides en mélange avec des peptides supérieurs et inférieurs | 2 | 5,2 ± 0,6 (25 %) | (37 %) | |
| | 3 | 4,3 ± 0,6 (18 %) | 5,3 (55 %) | |
| | 4 | 3,8 ± 0,6 (12 %) | 5,0 (67 %) | |
| Ces fractions contiennent presque exclusivement des tripeptides et des dipeptides | 5 | 3,0 ± 0,6 (8 %) | | |
| | 6 | 2,6 ± 0,5 (11 %) | | |
| | 7 | 2,4 ± 0,5 (14 %) | | |

7

## TABLEAU IV

Hydrolyse de la caséine par l'Alcalase®, la Neutrase® et la PEM®

| : Etalonnage avec peptides<br>: synthétiques purs | : N°<br>: fraction | : TAILLES MOYENNES ET TENEURS | | |
|---|---|---|---|---|
| | | : Pour la fraction | : Cumuls | : |
| : Cette fraction ne con-<br>: tient aucun tripeptide<br>: et aucun dipeptide | : 1 | : $9,0 \pm 0,9$ (10 %) | : | : : |
| : Ces fractions contien-<br>: nent en majorité des té-<br>: trapeptides en mélange<br>: avec des peptides supé-<br>: rieurs et inférieurs | : 2<br>: 3<br>: 4 | : $5,0 \pm 0,6$ (18 %)<br>: $4,5 \pm 0,6$ (17 %)<br>: $3,9 \pm 0,6$ (16 %) | : 6,0<br>:(28 %):<br>: 5,3 (55 %):<br>: 5,0 (60%) | : :<br>: :<br>: :<br>: |
| : Ces fractions contien-<br>: nent presque exclusive-<br>: ment des tripeptides et<br>: des dipeptides | : 5<br>: 6<br>: 7 | : $3,6 \pm 0,6$ (11 %)<br>: $2,8 \pm 0,5$ (10 %)<br>: $2,4 \pm 0,5$ (19 %) | : | |

On illustre l'invention par des exemples qui suivent.

EXEMPLE 1

1. Dénaturation et hydrolyse :

600 kg de blanc d'oeuf atomisé sont mis en suspension dans 4 000 litres d'eau distillée. Le pH est ajusté à 6 avec l'acide phosphorique, . La cuve est amenée sous agitation à 105°C sous 1 bar d'azote en 40 minutes. Cette température est maintenue pendant 1 heure puis la suspension est refroidie sous agitation jusqu'à 45°C. Le pH est ajusté à 8,00 avec une suspension de chaux à 25 %. On ajoute alors successivement :
- 6 kg de P.E.M.®,
- 60 litres de Neutrase ® et
- 60 litres d'Alcalase®.

On maintient la température à 47°C pendant 5 heures et le pH à 7,8.

8

2. Séparation solide-liquide :

Le pH est ajusté à 7 avec l'acide phosphorique. La température est alors amenée à 100°C pendant 1 heure. L'insoluble est séparé sur filtre rotatif, sous vide, en présence de Dicalite® 4146 de la firme Dicalite.

3. Décoloration :

A la phase liquide obtenue au stade précédent, on ajoute par m³. 20 kg de charbon actif Norit ® Extra SX. Le traitement s'effectue sous atmosphère d'azote pendant 1 heure à 70°C.

Le charbon actif est ensuite éliminé sur filtre à mailles métalliques verticales en présence de Dicalite® 4146 de la firme Dicalite. Cette opération est effectuée à 50°C.

4. Ultrafiltration :

Le filtrat obtenu au stade précédent est soumis à l'ultrafiltration utilisant l'appareil PLEIADE de la société RHONE-POULENC avec membranes Iris® 3038 ayant un point de coupure de 15 000 daltons ; cette opération est effectuée à une température de 45°C.

L'ultrafiltrat ainsi obtenu peut être utilisé comme matière première pour la fabrication d'un aliment entéral, soit tel quel, soit après dilution ou concentration sur évaporateur tubulaire ou à plaques, soit encore après séchage par atomisation.

Le tableau V donne les caractéristiques d'un hydrolysat à 15 grammes d'azote/litre.

Le tableau VI présente les aminoacidogrammes du blanc d'oeuf de départ et de l'ultrafiltrat.

## TABLEAU V

Composition et caractéristiques du produit obtenu selon l'invention.

(Hydrolysat d'ovalbumine)

|  | Hydrolysat ultrafiltré |
| --- | --- |
| Azote total | 15 g/l |
| Osmolarité | 900 mOs/kg |
| pH | 7,0 |
| Sodium | 150 meq/l |
| Potassium | 40 meq/l |
| Calcium | 550 mg/l |
| Chlore | 20 meq/l |
| Phosphore total | 18 mg/l |
|  |  |
| Répartition des protides |  |
|  |  |
| Aminoacides libres | 11 % |
| Dipeptides | 20,5 % |
| Tripeptides | 20,5 % |
| Tétrapeptides | 20,0 % |
| Teneur en di-, tri- peptides | 41 % |
| Teneur en di-, tri- tétra- peptides | 61 % |
| Oligopeptides (supérieurs aux tétra- peptides) | 28 % |

TABLEAU VI

| Composition en aminoacides de la matière première et de l'hydrolysat ultrafiltré d'ovalbumine. (résidus en pour cent) | | |
|---|---|---|
| | Matière première | Hydrolysat ultrafiltré |
| Acide aspartique et asparagine | 10,1 | 11,2 |
| Thréonine | 5,4 | 5,6 |
| Sérine | 7,6 | 8,7 |
| Acide glutamique et glutamine | 12,1 | 12,6 |
| Proline | 3,7 | 6,6 |
| Glycine | 6,9 | 6,6 |
| Alanine | 9,1 | 9,7 |
| Cystéine | 1,9 | 2,0 |
| Valine | 7,1 | 7,5 |
| Méthionine | 4,0 | 3,3 |
| Isoleucine | 4,2 | 4,3 |
| Leucine | 8,1 | 7,7 |
| Tyrosine | 2,8 | 2,1 |
| Phénylalanine | 4,2 | 3,5 |
| Lysine | 5,9 | 6,0 |
| Histidine | 1,8 | 1,8 |
| Tryptophane | 0,9 | 0,7 |
| Arginine | 4,2 | 4,0 |

EXEMPLE 2

1. Hydrolyse :

1500 kg de caséine lactique sont mis en suspension dans 10 000 litres d'eau. Le pH de la suspension est ajusté à 9,0 par addition d'une solution 5N de soude ou de potasse, dont la composition est déterminée par la teneur en sodium ou en potassium souhaitée dans le produit final.

On introduit successivement, à 5 minutes d'intervalle l'Alcalase ® , la Neutrase ® et la PEM® à la concentration indiquée sur le tableau I. Le pH est maintenu à 8 par addition de la solution d'alcali pendant la première heure, ensuite on laisse le pH évoluer librement vers 7,6. La durée de l'hydrolyse est fixée à 270 minutes.

2. Destruction de l'activité enzymatique - chauffage - refroidissement -

Le produit d'hydrolyse est porté à 95-98°C par passage sur échangeur à plaques contre la vapeur. Le produit est maintenu à la même température pendant 15 minutes.

Le produit est ensuite refroidi par passage sur échangeur à plaques contre l'eau froide à 15°C jusqu'à une température de 50°C.

3. Ultrafiltration :

L'ultrafiltration de l'hydrolysat refroidi est réalisée sur appareil PLEIADE de la société RHONE-POULENC, à une température de 49°C. Les membranes d'ultrafiltration sont de la société RHONE-POULENC et référencées Iris 3038, présentant un point de coupure de 15 000 daltons.

Comme pour l'exemple 1, l'ultrafiltrat peut être ensuite soit dilué, soit concentré.

Le tableau VII donne les caractéristiques d'un hydrolysat à 11 grammes d'azote/l et le tableau VIII les aminoacidogrammes de la caséine lactique de départ et de l'ultrafiltrat obtenu selon l'invention.

## TABLEAU VII

Composition et caractéristiques du produit obtenu selon l'invention.

(Hydrolysat de caséine)

|  | Hydrolysat ultrafiltré |
|---|---|
| Azote total | 11 g/l |
| Osmolarité | 490 mOs/kg |
| pH | 7,6 |
| Sodium | 80 meq/l |
| Potassium | 35 meq/l |
| Calcium | 10 meq/l |
| Ammoniaque | 25 meq/l |
| Chlore | 25 meq/l |
| Phosphore total | 0,8 mg/l |
|  |  |
| Répartition des protides |  |
|  |  |
| Aminoacides libres | 7 % en poids |
| Dipeptides | 25 % |
| Tripeptides | 25 % |
| Tétrapeptides | 25 % |
| Teneur en di-, tri- peptides | 50 % |
| Teneur en di-, tri- tétra- peptides | 75 % |
| Oligopeptides (supérieurs aux tétrapeptides) | 18 % |

TABLEAU VIII

| Composition en aminoacides de la matière première et de l'hydrolysat ultrafiltré de la caséine obtenu selon l'invention. (Résidus en pour cent) | | |
|---|---|---|
| | Matière première | Hydrolysat ultrafiltré |
| Acide aspartique et asparagine | 6,6 | 7,0 |
| Thréonine | 5,6 | 5,7 |
| Sérine | 7,2 | 6,8 |
| Acide glutamique et glutamine | 20,8 | 19,4 |
| Proline | 12,3 | 12,0 |
| Glycine | 3,0 | 2,9 |
| Alanine | 4,0 | 4,2 |
| Cystéine | 0,5 | 2,9 |
| Valine | 6,9 | 6,6 |
| Méthionine | 1,3 | 1,3 |
| Isoleucine | 2,7 | 3,0 |
| Leucine | 8,6 | 8,3 |
| Tyrosine | 3,7 | 3,4 |
| Phénylalanine | 4,1 | 3,9 |
| Lysine | 7,4 | 7,3 |
| Histidine | 2,4 | 2,4 |
| Tryptophane | non déterminé | - |
| Arginine | 2,9 | 2,9 |

EXEMPLE 3

Cet exemple est relatif à la présentation pharmaceutique d'un aliment diététique pour alimentation entérale, prêt à l'emploi.

A l'hydrolysat ultrafiltré obtenu selon l'exemple 1 ou 2, sont ajoutés des hydrates de carbone sous forme de maltodextrines, des lipides sous forme d'huiles végétales, des vitamines, des sels minéraux, des oligoéléments ainsi que plusieurs additifs technologiques nécessaires à la tenue en émulsion du produit fini. L'aliment entéral ainsi constitué est stérilisé par la technique UHT (Ultra Haute Température), puis il est réparti sous un volume compris entre 200 et 1000 ml, en emballages métalliques, ou emballages cartonnés.

A titre d'exemple, une formule à 4180 kJ/l (soit 1000 kcal/l) ayant une osmolarité comprise entre 300 et 450 mOs/kg présente la composition suivante :

| - hydrolysat obtenu selon l'invention | quantité d'hydrolysat pour faire 21 g de matière sèche |
|---|---|
| - maltodextrines | 60 g |
| - amidon | 10 g |
| - mélange lipidique (huile de soja, colza, onagre) | 8 g |
| - triglycérines à chaines moyennes | 8,7 g |
| - mélange vitaminique | 0,1 g |
| - sulfate de magnésium | 0,7 g |
| - chlorure de calcium | 0,2 g |
| - glycérophosphate de calcium | 0,8 g |
| - sulfate de fer (ferreux) | 0,01 g |
| - carbonate de manganèse | 0,003 g |
| - sulfate de zinc | 0,008 g |
| - sulfate de cuivre | 0,002 g |
| - iodure de potassium | 0,05 g |
| - émulsifiant | 1,3 g |
| - antioxydant des huiles | 0,003 g |
| - eau distillée q.s.p. | 500 ml |

EXEMPLE 4

Cet exemple est relatif à la présentation pharmaceutique d'un aliment pour alimentation entérale, sous forme de poudre à diluer dans l'eau avant l'emploi (500 Kcal/500 ml).

A 21 g d'hydrolysat séché par atomisation, on ajoute des maltodextrines, des lipides, des oligoéléments et des vitamines ainsi que les additifs technologiques nécessaires à la bonne mise en émulsion de la poudre dans l'eau. La composition pour un sachet à mettre en suspension est identique à celle présentée dans l'exemple 3, mais ne contient pas d'eau à l'exception de l'humidité résiduelle de l'ordre de 3 %.

**Revendications**

1. Procédé de fabrication d'un hydrolysat enzymatique de protéines riche en di- et tri-peptides, et ayant une teneur réduite en aminoacides libres, utilisable notamment en nutrition artificielle et en diététique, ledit procédé étant caractérisé en ce qu'il comporte une étape d'hydrolyse enzymatique d'une protéine, utilisant une association de trois enzymes suivantes, ajoutées simultanément ou successivement :

   a) une protéase bactérienne utilisable en milieu voisin de la neutralité (pH 5 à 8) généralement extraite de souches de Bacillus subtilis, choisie dans le groupe comprenant la Neutrase®, la Protéase B500® et la H.T. Protéolytique®,

   b) une protéase bactérienne utilisable en milieu alcalin (pH 7 à 11) extraite de souches de Bacillus licheniformis ou de Bacillus subtilis, choisie dans le groupe comprenant l'Alcalase®, la Protéase $A_2$® et l'Optimase®, et

   c) une enzyme pancréatique d'origine animale, choisie dans le groupe comprenant la trypsine ou un concentré de trypsine ou le produit commercialisé sous le nom de PEM®,

   dans les conditions d'hydrolyse suivantes :
   - un pH neutre à alcalin de 7 à 10,
   - une température de 20 à 70°C, de préférence de 30 à 50°C,
   - une durée d'hydrolyse égale ou inférieure à 300 minutes.

2. Procédé selon la revendication 1, caractérisé en ce que l'association d'enzymes comprend la Neutrase®, l'Alcalase® et la PEM®.

3. Procédé selon la revendication 1 ou 2, caractérisé en ce que la concentration en protéines de la suspension de matière protéique à hydrolyser est de 5 à 20 % en poids par volume.

4. Procédé selon la revendication 2 ou 3, caractérisé en ce que le rapport enzyme/substrat est de 5 = 10 % pour l'Alcalase® ou la Neutrase® et de 0,5-1 % pour la PEM®.

**5.** Procédé selon l'une quelconque des revendications 1 à 4, caractérisé en ce que la protéine de départ est du blanc d'oeuf ou de la caséine.

**6.** Procédé selon l'une quelconque des revendications 1 à 5, caractérisé en ce que l'hydrolysat enzymatique de protéines obtenu est inactivé par chauffage à 95-100°C pendant 10 à 15 minutes.

**7.** Procédé selon la revendication 6, caractérisé en ce que l'hydrolysat enzymatique inactivé est soumis à une ultrafiltration.

**Claims**

**1.** Process for the manufacture of an enzymatic protein hydrolyzate rich in dipeptides and tripeptides and having a reduced free amino acid content, which can be used especially in artificial nutrition and in dietetics, the said process being characterized in that it comprises a step for the enzymatic hydrolysis of a protein using a combination of the following three enzymes, added simultaneously or successively:
a) a bacterial protease which can be used in a medium at an approximately neutral pH (pH 5 to 8), generally extracted from strains of Bacillus subtilis and selected from the group comprising Neutrase®, Protease B500® and H.T. Proteolytic®,
b) a bacterial protease which can be used in an alkaline medium (pH 7 to 11), extracted from strains of Bacillus licheniformis or Bacillus subtilis and selected from the group comprising Alcalase®, Protease A$_2$® and Optimase®, and
c) a pancreatic enzyme of animal origin, including trypsin or a trypsin concentrate or the product marketed under the name PEM®, under the following hydrolysis conditions:
- a neutral to alkaline pH of 7 to 10,
- a temperature of 20 to 70°C, preferably 30 to 50°C, and
- a hydrolysis time preferably equal to or less than 300 minutes.

**2.** Process according to claim 1, characterized in that the enzyme combination comprises Neutrase®, Alcalase® and PEM®.

**3.** Process according to claim 1 or 2, characterized in that the protein concentration of the suspension of protein substance to be hydrolyzed is 5 to 20% by weight/volume.

**4.** Process according to claim 2 or 3, characterized in that the enzyme/substrate ratio is 5-10% for Alcalase® or Neutrase® and 0.5-1% for PEM®.

**5.** Process according to any one of claims 1 to 4, characterized in that the starting protein is egg white or casein.

**6.** Process according to any one of claims 1 to 5, characterized in that the enzymatic protein hydrolyzate obtained is inactivated by heating at 95-100°C for 10 to 15 minutes.

**7.** Process according to claim 6, characterized in that the inactivated enzymatic hydrolyzate is subjected to ultrafiltration.

**Patentansprüche**

**1.** Verfahren zur Herstellung eines enzymatischen, di- und tri-peptidreichen Proteinhydrolysats mit einem verringerten Gehalt an freien Aminosäuren, das insbesondere zur künstlichen Ernährung sowie in der Diätetik verwendbar ist,
**dadurch gekennzeichnet,** daß
es eine Stufe der enzymatischen Hydrolyse eines Proteins unter Verwendung einer Kombination aus den drei nachstehenden Enzymen umfaßt, die gleichzeitig oder nacheinander zugegeben werden:
a) Einer bakteriellen Protease, die in einem nahezu neutralen Milieu (pH 5 - 8) verwendbar ist und allgemein aus Stämmen von Bacillus subtilis extrahiert und ausgewählt ist unter Neutrase®, Protease B500® und H.T. Protéolytique®,
b) einer bakteriellen Protease, die in alkalischem Milieu (pH 7 - 11) verwendbar ist und aus Stämmen von Bacillus licheniformis oder Bacillus subtilis extrahiert und ausgewählt ist unter

Alcalase®, Protease A$_2$® und Optimase®,

und

c) einem Pancreasenzym tierischen Ursprungs, das unter Trypsin und Trypsinkonzentraten sowie dem unter der Bezeichnung PEM® kommerzialisierten Produkt ausgewählt ist,

unter folgenden Hydrolysebedingungen:

- neutraler bis alkalischer pH-Wert im Bereich von 7 bis 10,
- Temperatur im Bereich von 20 - 70 ° C und vorzugsweise von 30 bis 50 ° C,
- Hydrolysedauer gleich oder kleiner als 300 min.

2. Verfahren nach Anspruch 1, dadurch gekennzeichnet, daß die Enzymkombination Neutrase®, Alcalase® und PEM® enthält.

3. Verfahren nach Anspruch 1 oder 2, dadurch gekennzeichnet, daß die Proteinkonzentration der Suspension der zu hydrolysierenden Proteinsubstanz 5 bis 20 % (Gew./Vol.) beträgt.

4. Verfahren nach Anspruch 2 oder 3, dadurch gekennzeichnet, daß das Verhältnis Enzym/Substrat bei Alcalase® oder Neutrase® 5 bis 10 % und bei PEM® 0,5 - 1 % beträgt.

5. Verfahren nach einem der Ansprüche 1 bis 4, dadurch gekennzeichnet, daß das Ausgangsprotein Eiweiß oder Casein ist.

6. Verfahren nach einem der Ansprüche 1 bis 5, dadurch gekennzeichnet, daß das erhaltene enzymatische Proteinhydrolysat durch 10 bis 15 min Erhitzen auf 95 bis 100 ° C inaktiviert wird.

7. Verfahren nach Anspruch 6, dadurch gekennzeichnet, daß das inaktivierte enzymatische Hydrolysat einer Ultrafiltration unterzogen wird.